# FASCICULE DE BREVET EUROPEEN

(11) **EP 2 703 373 B1**
(45) Date de publication et mention de la délivrance du brevet: **25.10.2017**
(21) Numéro de dépôt: 13305742.2
(22) Date de dépôt: 04.06.2013
(51) Int. Cl.: C07C 7/04, C07C 11/107

(54) **Procede de separation de l'hexene-1 a partir d'un melange de produits issus d'une zone de trimerisation de l'ethylene**
Separationsverfahren von Hexen-1 aus einer Produktmischung, die aus einer Ethylentrimerisationszone stammt
Method for separating the hexene-1 from a mixture of products taken from an ethylene trimerisation area

(30) Priorité: 04.07.2012 FR 1201886
(43) Date de publication de la demande: 05.03.2014
(73) Titulaire: AXENS, 92508 Rueil Malmaison Cedex (FR)
(72) Inventeur: Vinel, Daniel-Jean, 78130 LES MUREAUX (FR); Chodorge, Jean-Alain, 92160 ANTONY (FR); Pigourier, Jérôme, 92190 MEUDON (FR); Martin, Pierre-Yves, 92500 RUEIL MALMAISON (FR); Bournay, Laurent, 69440 CHAUSSAN (FR)
(74) Mandataire: Schmitt, Nicolas A.J.

(56) Documents cités:
- EP-A1- 2 098 542
- WO-A1-2011/112184

## Description

### Domaine de l'invention

La présente invention concerne la production de l'hexène-1, plus précisément, un procédé de production et de séparation de l'hexène-1 à partir d'un mélange de produits issus d'une zone de trimérisation de l'éthylène. Une unité de production et de séparation de l'hexène-1 comprenant une zone de trimérisation de l'éthylene est également divulguée.

### Art antérieur

L'hexène-1 peut être produite avec une sélectivité élevée par oligomérisation de l'éthylène notamment par trimérisation de l'éthylène dans un système catalytique, en phase généralement homogène, en présence ou non de solvant. En dépit de la sélectivité du catalyseur de trimérisation pour la formation de l'hexène-1, la réaction forme, en dehors de l'éthylène non converti, des produits secondaires. L'effluent issu de la trimérisation de l'éthylène peut comprendre des alpha-oléfines, du butène-1, de l'octène-1, des décènes et principalement de l'hexène-1, ainsi que le solvant de l'oligomérisation et les sous-produits C12+ (c'est-à-dire comportant au moins 12 atomes de carbone par molécule). Ledit effluent comprend aussi du catalyseur.

Un des problèmes majeurs dans ce domaine concerne la séparation de l'hexène-1 des autres constituants du mélange issu de la réaction de trimérisation de l'éthylène avec un degré de pureté conforme à l'utilisation ultérieure de l'hexène-1, par exemple, comme co-monomère dans la fabrication de polyéthylène.

Un certain nombre de procédures ont été élaborées pour améliorer la séparation de l'hexène-1 tout en réduisant les coûts liés à l'investissement nécessaire pour construire les installations mises en oeuvre.

Typiquement, la séparation des produits d'oligomérisation peut être réalisée avec des moyens de séparation, tels que les colonnes de distillation, fonctionnant en série et basées sur les différences de points d'ébullition des composés à séparer. Ainsi, la première colonne de distillation sépare l'éthylène, composé le plus volatil qui sort en haut de la colonne, des autres constituants plus lourds sortant en fond de colonne et alimentent une seconde colonne dans laquelle de l'hexène-1 est obtenu.

Par exemple, le brevet US 7,476,775 décrit un procédé de séparation d'un effluent d'oligomérisation comprenant une séparation de l'effluent issu de la réaction d'oligomérisation en une fraction liquide et une fraction gazeuse, la distillation de la fraction liquide et de la fraction gazeuse pour récupérer des fractions de produits d'oligomérisation.

Le brevet US 5,853,551 de la demanderesse décrit un schéma de séparation de l'éthylène et de butène-1 des C4+, puis du butène-1 des C6+, puis de l'hexène et l'octène des C10+ et du solvant, et enfin deux séparations d'une part de l'hexène-1 et de l'octène-1 et d'autre part du solvant et des C10+. Le brevet US 5,853,551 décrit un schéma consistant à ajouter du butène-1 supplémentaire dans la première colonne à distiller afin de maintenir la température en fond de colonne à des valeurs autour desquelles les alpha-oléfines sont stables et de pouvoir séparer l'éthylène avec un peu de butène-1 en tête de colonne.

Le brevet EP 2 098 542 décrit un procédé de séparation d'hexène-1 issu d'un effluent de trimérisation d'éthylène dans lequel le fond de colonne de distillation est recyclé vers la zone réactionnelle.

La demande WO 2011/112184 décrit le même type de procédé dans lequel le fond d'une colonne d'évaporation flash est recyclé afin d'aider à rebouillir ladite colonne. Les procédés proposés dans l'art antérieur souffrent d'insuffisance notamment en termes d'efficacité dans la sélectivité de la séparation et d'efficacité énergétique. Il existe donc toujours un besoin d'amélioration des procédés de séparation des produits d'oligomérisation, notamment de trimérisation de l'éthylène pour la production d'hexène-1.

Il a été mis au point par la demanderesse, un nouveau procédé de séparation des produits d'oligomérisation permettant de pallier les problèmes cités plus haut.

### Résumé de l'invention

La présente invention concerne un procédé de séparation de l'hexène-1 à partir d'un mélange issu d'une section réactionnelle de trimérisation de l'éthylène, ledit mélange comprenant, l'éthylène, le solvant, le catalyseur de la trimérisation de l'éthylène et les produits formés dont l'hexène-1, le procédé comprenant au moins les étapes suivantes:
a) on sépare dans une première colonne de distillation, le mélange issu de la réaction de trimérisation de l'éthylène, en une fraction de tête comprenant l'éthylène et une fraction de fond,
b) on sépare dans au moins une autre colonne de distillation au moins une partie d'un l'effluent provenant de la fraction de fond issue de l'étape a) en une fraction de tête comprenant de l'hexène-1 et du butène-1 et une fraction de fond,
c) on sépare dans une colonne de distillation finale au moins une partie de la fraction comprenant de l'hexène-1 et du butène-1 issue de l'étape b) en une fraction de tête comprenant principalement du butène-1 et en une fraction de fond comprenant principalement de l'hexène-1 et dans ledit procédé :
   - au moins une partie d'un effluent provenant de la fraction de fond issue de l'étape b) est renvoyée dans la section réactionnelle et au moins une autre partie de ladite fraction de fond issue de l'étape b) est utilisée dans au moins une boucle de recirculation reliant la section réactionnelle et la colonne de ladite étape b), ladite boucle de recirculation permettant de refroidir la section réactionnelle et de rebouillir ladite colonne de l'étape b).

Dans une variante selon l'invention, au moins une partie d'un effluent provenant de la fraction de fond issue de l'étape b) est envoyée dans au moins une colonne de distillation pour en éliminer une fraction de fond comprenant essentiellement des oléfines en C8+ avant de renvoyer au moins une partie de la fraction de tête issue de ladite colonne de distillation dans la section réactionnelle.

Dans une autre variante selon l'invention, au moins une partie d'un effluent provenant de la fraction de fond issue de l'étape a) est envoyée dans au moins une colonne de distillation pour en éliminer une fraction de fond comprenant essentiellement des oléfines en C8+ avant d'envoyer au moins une partie de la fraction de tête issue de ladite colonne de distillation dans la colonne de distillation de l'étape b).

De manière avantageuse selon le procédé de l'invention, on renvoie au moins une partie d'un effluent provenant de la fraction de fond issue de l'étape b) en tête de la première colonne de l'étape a) pour laver l'éthylène sortant en tête de ladite première colonne et entrainer la fraction d'oléfines en C4⁺ vers le fond de ladite première colonne.

D'autres caractéristiques et avantages de l'invention apparaîtront à la lecture de la description détaillée qui suit.

### Description détaillée de l'invention

### Liste des figures.

La description et les figures présentées ci-après permettent d'illustrer plus clairement les modes de réalisation du procédé de l'invention et font apparaître l'ensemble des avantages associés à la mise en oeuvre de ce procédé.

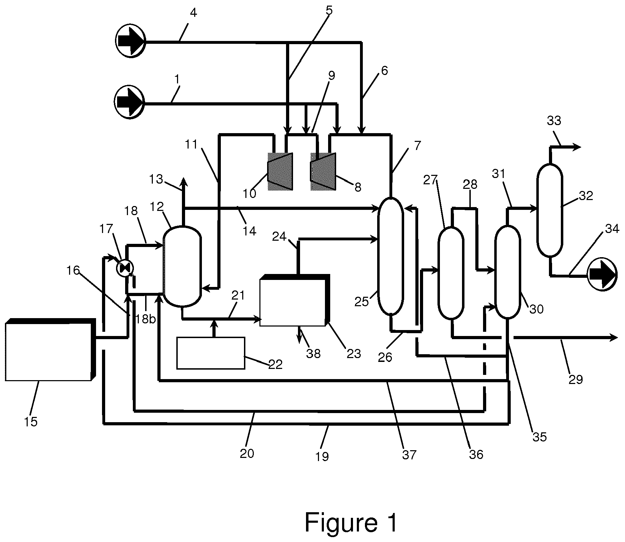

La Figure 1 illustre, de manière non limitative, un mode de réalisation, dans lequel au moins une partie de la fraction de fond issue de l'étape a) est envoyée dans une colonne de distillation pour en éliminer une fraction en C8+ avant d'être envoyée dans la colonne de distillation de l'étape b).

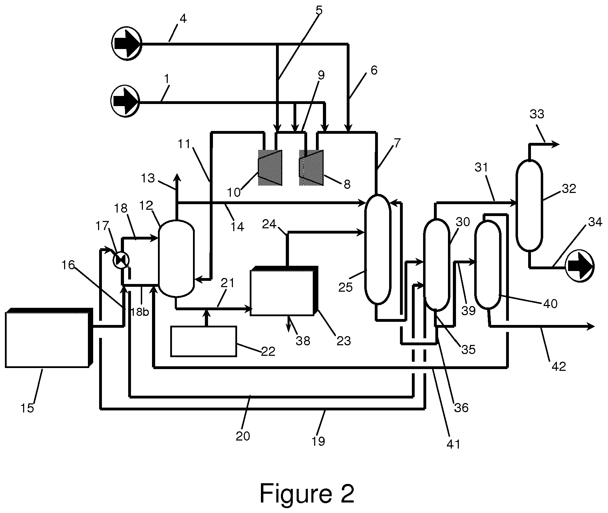

La figure 2, illustre de manière non limitative, un mode de réalisation, dans lequel au moins une partie d'un effluent provenant de la fraction de fond issue de l'étape b) est envoyée dans au moins une colonne de distillation pour en éliminer une fraction de fond comprenant essentiellement des oléfines en C8+ avant de renvoyer au moins une partie de la fraction de tête issue de ladite colonne de distillation dans la section réactionnelle.

Dans la suite du texte, il sera fait référence aux numéros indiqués dans les figures 1 et 2 pour plus de clarté.

### Section réactionnelle

Le procédé de l'invention comprend une section réactionnelle de trimérisation de l'éthylène. Lors de la réaction de trimérisation, on met en contact de l'éthylène avec un catalyseur d'oligomérisation pour produire de l'hexène-1.

Les catalyseurs permettant de réaliser la réaction de trimérisation de l'éthylène peuvent être hétérogènes ou homogènes par rapport au milieu réactionnel. Ils comportent généralement au moins l'un des éléments suivants : un métal de transition, un composé acide ou un composé basique.

Généralement les différents composés sont donc séparés en deux parties, d'une part le constituant essentiel du catalyseur activé qui contient le métal principal, donc le plus souvent appelé « catalyseur », et les constituants secondaires qui servent à activer le constituant essentiel et maintenir son activité, donc le plus souvent appelés « activateur ».

Avantageusement selon le procédé de l'invention, les différents composés intervenant dans la préparation du catalyseur activé sont stockés dans au moins deux ballons différents et le catalyseur résultant de leur mélange est stocké dans un ballon. Dans un mode de réalisation du procédé selon l'invention, le catalyseur activé déjà prêt à l'emploi est transféré dans un ballon unique à partir duquel on alimente le réacteur. Le catalyseur et l'activateur sont avantageusement pompés séparément avec des débits controlés afin d'ajuster les rapports molaires entre ces différents composés. La réaction de trimérisation de l'éthylène est exothermique. Il est donc nécessaire de contrôler la chaleur de réaction. Avantageusement, ce contrôle de l'exothermicité est réalisé au moyen d'une ou plusieurs boucles de recyclage du milieu réactionnel par passage dudit milieu réactionnel à travers au moins un échangeur de chaleur. Ainsi, le catalyseur et l'activateur peuvent être injectés dans les boucles de recyclage du réacteur de la section réactionnelle, de préférence à deux endroits différents.

Tout catalyseur de trimérisation de l'éthylène peut être utilisé dans le schéma de procédé selon l'invention. En particulier, les catalyseurs décrits dans la demande de brevet WO07/039851, ou dans les demandes de brevets WO04/056479, WO03/053891 ou WO02/04119. Le catalyseur utilisable dans le procédé selon l'invention peut comprendre un complexe d'un élément de transition. Ce complexe est formé par la complexation du métal avec au moins un ligand comprenant des hétéroatomes tels que l'azote, l'oxygène ou le phosphore, ce qui permet la solubilisation de ce métal en milieu hydrocarboné. Le complexe peut être par exemple un composé oxygéné organique : éther, ester, acétal, cétal, etc....

Il est connu que des catalyseurs à base de chrome sont des catalyseurs de trimérisation de l'éthylène très actifs qui conduisent à la formation d'hèxène-1, et ce avec une bonne sélectivité, c'est-à-dire avec moins de polyéthylène et une faible proportion de butènes. Des catalyseurs et activateurs utilisables dans le procédé selon l'invention sont par exemple des mélanges d'un composé du chrome (catalyseur) tel que les halogènures, les carboxylates, les acétylacétonates, les aryloxy ou alcoxy, avec un composé aryloxy d'un élément tel que l'aluminium, le magnésium, le calcium, le strontium ou le baryum, et un hydrocarbylaluminium (activateur). De tels catalyseurs et activateurs sont par exemple décrits dans le brevet FR 2802833. Le composé hydrocarbylaluminium mentionné ci-avant peut également être remplacé par tout composé hydrocarbyl d'un autre élément tel que l'aluminium, le gallium, niobium, le tantale l'yttrium ou une terre rare tel que le lanthane. Le composé hydrocarbyl peut également être halogéné, comme décrit par exemple dans la demande de brevet WO 2007/039851.

Selon l'invention, les composants du catalyseur activé sont avantageusement mis en contact dans un solvant composé d'au moins un hydrocarbure choisi parmi les hydrocarbures saturés suivants : l'hexane, le cyclohexane, l'heptane, le butane, le toluène, l'orthoxylène, l'isobutane, le 2,2 diméthylpentane ou parmi les hydrocarbures insaturés suivants : mono-oléfines ou les dioléfines comportant de 4 à 10 atomes de carbone ou les composés aromatiques tels que par exemple le benzène, le toluène ou les xylènes. On utilise de manière plus préférée des hydrocarbures insaturés.

Avantageusement, le solvant utilisé est choisi parmi le 2,2 diméthylpentane, le cyclohexane, le toluène ou l'orthoxylène, et plus préférentiellement parmi le 2,2 diméthylpentane et le cyclohexane.

Selon le procédé de l'invention, la réaction de trimérisation de l'éthylène est conduite dans un ou plusieurs réacteur(s). Le ou les réacteur(s) peuvent être tout moyen adapté connu de l'homme du métier. Par exemple, dans le cas de catalyseurs hétérogènes solides, on peut utiliser des réacteurs à lit fixe, des réacteurs à lit fluidisé, des réacteurs à lit circulant, des colonnes catalytiques ou des réacteurs adaptés à la mise en oeuvre de catalyseurs homogènes tels que des réacteurs tubulaires, à cuves agitées, à équilibre liquide/vapeur...

La réaction de trimérisation de l'éthylène peut être conduite en batch, en semi-continu ou en continu. De façon préférée, elle est conduite de manière continue.

Selon le procédé de l'invention, les conditions opératoires du réacteur de trimérisation sont telles que le milieu réactionnel est au point de bulle. Ce point de bulle est garanti par le soutirage permanent de vapeur, généralement un faible débit vapeur (13) en tête de réacteur. Avantageusement selon l'invention, au moins une partie de la vapeur soutirée en tête de réacteur (13) est envoyée (14) dans la colonne de distillation 25. De manière préférée, ledit réacteur est parfaitement agité. Cette agitation est favorisée entre autre par la boucle de recyclage vers le réacteur (18, 18b).

De manière avantageuse, la température de la réaction de trimérisation de l'éthylène est comprise entre 0 et 300 °C, de manière préférée entre 30 et 210 °C, de manière plus préférée entre 100°C et 180°C. La pression d'opération est choisie de manière à maintenir le milieu réactionnel de la réaction de trimérisation de l'éthylène au point de bulle. Ladite pression est avantageusement comprise entre 0,1 et 35 MPa, de manière préférée entre 1 et 20 MPa, et manière plus préférée entre 5 et 15 MPa.

Selon le procédé de l'invention, dès la sortie de la section réactionnelle de trimérisation de l'éthylène (réacteur 12), le catalyseur et son activateur contenus dans la fraction de fond (21) sortant dudit réacteur sont avantageusement neutralisés par l'injection d'un inhibiteur (22). Le mélange est généralement réalisé en utilisant un mélangeur dynamique en ligne ou d'un mélangeur statique en ligne. La neutralisation du catalyseur et de son activateur est réalisée par tout composé connu de l'homme du métier pour son activité inhibitrice du catalyseur de trimérisation. Ledit composé est généralement sélectionné parmi les amines, de préférence les amines primaires ou secondaires de formule générale R1 R2NH dans laquelle R1 est l'hydrogène ou un radical hydrocarboné et R2 est un radical hydrocarboné. On utilisera de manière préférée un inhibiteur sélectionné parmi les composés suivants ou leurs mélanges : cyclohexylamine, éthyl-2-hexylamine, arylamine, stéarylamine, oleylamine, aniline, N-methyl aniline, dibutylamine, didecylamine, les mélanges d'amines obtenus à partir de corps gras naturels tels que le suif, l'huile de palme ou l'huile de coprah.

L'inhibiteur peut également être sélectionné parmi les alcools linéaires ou ramifiés ayant de préférence de 2 à 20 atomes de carbones, de manière plus préférée de 5 à 15 atomes de carbone. De manière très préférée l'inhibiteur est un alcool ramifié comprenant au moins une ramification hydrocarbyl, par exemple un alcool comportant au moins une ramification méthyl, éthyl, propyl, ou butyl fixée sur une chaine alcool ayant de préférence entre 2 et 16 atomes de carbones et de manière très préférée entre 4 et 10 atomes de carbone. De manière préférée, l'inhibiteur est choisi parmi les alcools suivants ou leurs mélanges: 1-hexanol, 2-hexanol, 3-hexanol, 2-methyl-hexanol, 2-ethyl-hexanol, 3-octanol, 1-heptanol, 2-heptanol, 3-heptanol, 4-heptanol, 2-methyl-3-heptanol,1-octanol, 2-octanol, 3-octanol, 4-octanol, 7-méthyl-2décanol,1-decanol, 2-decanol, 3-decanol, 4-decanol, 5-decanol, 2 ethyl-1-decanol.

L'inhibiteur est de préférence ajouté à la température à laquelle a lieu la réaction de trimérisation.

Cette étape de neutralisation de toute activité catalytique permet notamment d'éviter que la présence de catalyseur encore actif ne vienne altérer, par réaction non-contrôlée, la pureté des produits de la réaction initiale de trimérisation.

Le milieu réactionnel issu du réacteur (21) neutralisé est ensuite envoyé dans une unité de séparation (23) dans laquelle on sépare d'une part, le catalyseur neutralisé ainsi que les sous-produits lourds (C12+) de la réaction et d'autre part l'éthylène non converti, les autres produits formés et le solvant. Selon l'invention, la fraction issue de l'unité de séparation (23) contenant l'éthylène non converti, les produits formés et le solvant est envoyée sous forme vapeur (24) vers la section de distillation. La séparation dans l'unité de séparation (23) est avantageusement réalisée par une série de flashs étagés.

Avantageusement, le milieu réactionnel issu du réacteur (21) est détendu à une pression comprise entre 2 et 5 MPa et de façon préférée entre 3 et 4,5 MPa. Ledit milieu réactionnel détendu est ensuite vaporisé par chauffage jusqu'à une température comprise entre 150 et 200°C et de manière préférée entre 160 et 190°C. Cette vaporisation est avantageusement effectuée dans un échangeur de chaleur. La phase vapeur est alors séparée de la phase liquide dans un ballon. La phase vapeur est envoyée vers la section de distillation et la phase liquide est envoyée vers un deuxième flash.

La phase liquide résultant du premier flash est détendu à une pression comprise entre 0,5 et 2 MPa et de façon préférée entre 0,8 et 1,5 MPa. Ladite phase liquide détendue est ensuite vaporisée par chauffage jusqu'à une température comprise entre 160 et 220°C et de manière préférée entre 170 et 210°C. Cette vaporisation est avantageusement effectuée dans un échangeur de chaleur. La phase vapeur est alors séparée de la phase liquide dans un ballon. La phase vapeur est envoyée vers la section de distillation et la phase liquide est envoyée vers un troisième flash.

La phase liquide résultant du deuxième flash est détendue à une pression comprise entre 0,1 et 0,5 MPa. Ladite phase liquide détendue est ensuite vaporisée par chauffage jusqu'à une température comprise entre 160 et 220°C. Cette vaporisation est avantageusement effectuée dans un échangeur de chaleur, de préférence à couche mince ou à film tombant.

Le catalyseur neutralisé, ainsi que les sous-produits lourds (C12+) séparés dans l'unité de séparation (23) sont pompés et envoyés (38) vers un incinérateur.

### Section de distillation

Typiquement, les oléfines oligomères issues de la trimérisation de l'éthylène possèdent un poids moléculaire plus important que l'éthylène n'ayant pas réagi. D'une façon générale, l'éthylène n'ayant pas réagi présente une température d'ébullition plus basse que celle des oligomères issus de la réaction de trimérisation y compris l'hexène-1.

Selon l'invention, tout moyen de séparation connu de l'homme du métier mettant à profit ces écarts de volatilité et de poids moléculaire entre les produits à séparer peut être mis en oeuvre. Avantageusement selon l'invention, les moyens de séparation mis en oeuvre sont des colonnes de distillation de tout type.

Selon le procédé de l'invention, la fraction issue de l'unité de séparation (23) à savoir: l'éthylène non converti, les produits formés et le solvant, est envoyée sous forme vapeur (24) vers la section de distillation.

La section de distillation comprend avantageusement au moins trois colonnes de distillation, de préférence au moins quatre colonnes de distillation. Selon une variante préférée de l'invention, la section de distillation comprend quatre colonnes de distillation.

### Etape a)

Selon l'invention, la fraction issue de l'unité de séparation (23) à savoir: l'éthylène non converti, les produits formés et le solvant, est soumise à une étape a) de séparation. Ladite fraction est envoyée sous forme vapeur (24) vers une première colonne de distillation (25) dans laquelle l'éthylène non converti est séparé dans une fraction de tête du reste des composés dans la fraction de fond.

De manière avantageuse selon l'invention, la colonne de distillation de l'étape a) de séparation est mise en oeuvre à une pression comprise entre 0,1 et 1,5 MPa, de préférence entre 0,5 et 1 MPa, une température de tête de colonne comprise entre 0 et 100°C, de préférence entre 40 et 80°C, et une température de fond de colonne comprise entre 100 et 300°C, de préférence entre 140 et 220°C. De manière préférée, l'éthylène séparé en tête de la colonne (25) est renvoyé vers la section réactionnelle 12.

Avantageusement selon le procédé de l'invention, le renvoi de l'éthylène séparé en tête de colonne de la colonne (25) est réalisé (7, 9, 11) en même temps qu'au moins l'alimentation en hydrogène (4) et en éthylène frais (1). Le mélange de l'éthylène séparé en tête de colonne de la colonne (25), de l'hydrogène et de l'éthylène frais est avantageusement réalisé dans au moins un compresseur de recycle (référencés 10 ou 8 dans les figures 1 et 2). Selon l'invention, le mélange est introduit dans le réacteur (12) en utilisant un distributeur afin d'assurer une bonne dispersion des goutelettes d'éthylène au sein du milieu réactionnel (non représenté dans les figures). L'éthylene peut également être introduit dans au moins une des boucles de recyclage du procédé de l'invention.

De manière préférée, afin d'éviter l'utilisation de frigories pour condenser la tête de colonne de la première colonne de l'étape a), au moins une partie de l'effluent provenant de la fraction de fond issue de l'étape b) est renvoyée en tête de ladite première colonne de l'étape a) pour laver l'éthylène sortant en tête de ladite première colonne et entrainer la fraction d'oléfines en C4+ vers le fond de ladite première colonne. En effet, la fraction de fond issue de l'étape b) est principalement composée de solvant (au moins 95 % poids de solvant). Ce solvant permet d'absorber préférentiellement les produits lourds et les entraîner vers le fond de la colonne.

### Étape b)

Selon l'invention, au moins une partie d'un effluent provenant de la fraction de fond issue de l'étape a) est ensuite soumise à une étape b) de séparation dans laquelle on sépare dans au moins une autre colonne de distillation (30) au moins une partie dudit effluent provenant de ladite fraction de fond issue de l'étape a) en une fraction de tête (31) comprenant de l'hexène-1 et du butène-1 et une fraction de fond. Ladite fraction de fond est principalement composée de solvant. Avantageusement, la teneur en solvant de cette fraction est supérieure ou égale à 95% poids, de préférence supérieure à 98% poids et de manière préférée supérieure ou égale à 99,5% poids.

De manière avantageuse selon l'invention, la colonne de distillation de l'étape b) de séparation est mise en oeuvre à une pression comprise entre 0 et 0,5 MPa, de préférence entre 0,01 et 0,3 MPa, une température de tête de colonne comprise entre 20 et 150°C, de préférence entre 40 et 100°C, et une température de fond de colonne comprise entre 50 et 200°C, de préférence entre 80 et 150 °C

Selon l'invention, au moins une partie d'un effluent provenant de la fraction de fond issue de l'étape b) est renvoyée (37) dans la section réactionnelle et au moins une autre partie de ladite fraction de fond issue de l'étape b) est utilisée dans une boucle de recirculation (19) entre la section réactionnelle et ladite autre colonne de ladite étape b) pour refroidir la section réactionnelle et rebouillir la colonne de ladite étape b). Avantageusement selon l'invention, ladite autre partie de ladite fraction de fond issue de l'étape b) est prélevée soit en fond de la colonne (30), soit via un soutirage latéral préférentiellement situé à proximité du fond de la colonne et de manière plus préférée via un soutirage latéral situé dans le quart inférieur de ladite colonne.

En effet, la réaction de trimérisation de l'éthylène est exothermique. La chaleur générée par la réaction doit être extraite pour éviter une élévation incontrôlée de la température du milieu réactionnel. Selon le niveau de température atteint, les conséquences sont une perte de la sélectivité par dégradation thermique du catalyseur et des produits de la réaction. Selon l'invention, la chaleur de réaction générée dans le réacteur (12) est extraite en utilisant au moins une boucle de recirculation par le biais d'au moins une partie de l'effluent provenant de la fraction de fond issue de l'étape b). La chaleur générée dans le réacteur est extraite par l'intermédiaire d'une boucle de recyclage (18, 18b) du réacteur. La boucle de recyclage du reacteur (18, 18b) réalisé par le biais d'échangeurs, préférentiellement de type kettle (17) transfère la chaleur générée dans le réacteur (12) à l'effluent provenant de la fraction de fond issue de l'étape b) provoquant la vaporisation dudit effluent provenant de ladite fraction de fond.

Selon l'invention, la partie de l'effluent provenant de ladite fraction de fond issue de l'étape b) vaporisée à la sortie de l'échangeur (17) est renvoyée (20) vers la colonne de distillation de l'étape b) (30) fermant la boucle de recirculation. Le renvoi de la fraction (20) vers la colonne de distillation de l'étape b) joue le rôle de rébouilleur latéral et permet d'alléger la charge du rebouilleur principal de la colonne de distillation de l'étape b) et en conséquence d'économiser de la vapeur.

Le contrôle de la pression de vaporisation de la fraction de fond issue de l'étape b) dans l'échangeur de la boucle de recirculation et de la boucle de recyclage du réacteur permet d'ajuster l'écart de température moyen entre les flux chaud et froid et donc la capacité d'échange des échangeurs. On utilise avantageusement un échangeur de type kettle.

Un autre avantage de la boucle de recirculation est de permettre le contrôle de l'exothermicité de la réaction de trimérisation dans le réacteur (12), mais également de garantir que ledit réacteur soit parfaitement agité. La boucle de recirculation, permet d'assurer à la fois :
- l'évacuation de la chaleur dégagée par la réaction, qui, dans l'art antérieur est réalisée par exemple par la vaporisation d'eau dans un échangeur de type kettle. Cette vapeur d'eau générée possède une pression trop basse pour être efficacement utilisée dans le procédé. La vapeur d'eau ainsi produite doit donc être condensée dans un échangeur supplémentaire (par exemple un échangeur à air) pour être recyclée vers l'échangeur de type kettle. Le refroidissement de cette vapeur représente une consommation énergétique importante et l'installation associée (échangeur, ballon, pompe) représente un coût d'investissement additionnel.
- la majeure partie du rebouillage du fond de la colonne (30) de l'étape b), qui, dans l'art antérieur est réalisé par exemple grâce à un échangeur utilisant par exemple de la vapeur d'eau comme fluide chaud. La production de cette vapeur représente une consommation énergétique importante et l'installation associée à sa production (chaudière, pompe, traitement de l'eau) représente un coût d'investissement également important.

Selon l'invention, les quantités de chaleur à échanger pour le refroidissement du milieu réactionnel et la vaporisation d'une fraction de fond de la colonne de l'étape b) pour son rebouillage sont sensiblement similaires, ce qui présente l'avantage d'une intégration thermique efficace.

Le procédé selon l'invention présente l'avantage d'un gain d'une part énergétique car il consomme moins de vapeur, d'autre part un gain économique par une diminution des coûts d'installation.

Afin d'éviter une accumulation des hydrocarbures C8+ dans l'unité, il est préférable de les extraire à l'aide d'une colonne de distillation supplémentaire, qui peut être incorporée dans le schéma de procédé en au moins deux endroits différents.

Dans une variante préférée du procédé selon l'invention telle que illustré à la figure 1, au moins une partie d'un effluent provenant de la fraction de fond issue de l'étape a) est envoyée dans au moins une colonne de distillation (27) pour en éliminer une fraction de fond (29) comprenant essentiellement des oléfines en C8+ avant d'envoyer la fraction de tête (28) issue de ladite colonne de distillation dans la colonne de distillation de l'étape b) (colonne 30). De manière avantageuse selon l'invention, ladite colonne de distillation est mise en oeuvre à une pression comprise entre 0 et 1 MPa, de préférence entre 0,1 et 0,5 MPa, une température de tête de colonne comprise entre 60 et 160°C, de préférence entre 90 et 130°C, et une température de fond de colonne comprise entre 100 et 300°C, de préférence entre 170 et 240°C. Selon cette variante, le catalyseur frais (16) et au moins une partie de l'effluent provenant de la fraction de fond issue de l'étape b) (37) sont mélangés dans la boucle de recyclage du réacteur (18b) avec le liquide pompé du réacteur (12). Le flux 16 et le flux 37 sont introduits du coté « tube » de l'échangeur. Selon l'invention, au moins une autre partie de ladite fraction de fond issue de l'étape b) correspondant celle-ci à la boucle de recirculation est introduite du coté "calandre" de l'échangeur.

Dans une autre variante de réalisation du procédé selon l'invention telle que illustrée à la figure 2, au moins une partie d'un effluent provenant de la fraction de fond issue de l'étape b) (colonne 30) est envoyée dans au moins une colonne de distillation (colonne 40) pour en éliminer une fraction de fond comprenant essentiellement des oléfines en C8+ avant d'envoyer la fraction de tête (41) issue de ladite colonne de distillation dans la section réactionnelle. Selon cette variante, le catalyseur frais (16) et la fraction de tête (41) sont avantageusement mélangés dans la boucle de recyclage du réacteur (18b) avec le liquide pompé du réacteur (12) et introduits du coté « tube » de l'échangeur (17). De manière avantageuse selon l'invention, ladite colonne de distillation est mise en oeuvre à une pression comprise entre 0,05 et 1 MPa, de préférence entre 0,1 et 0,5 MPa, une température de tête de colonne comprise entre 70 et 150°C, de préférence entre 90 et 130 °C, et une température de fond de colonne comprise entre 100 et 300°C, de préférence entre 150 et 220 °C. Ladite fraction de tête issue de ladite colonne de distillation est principalement composée de solvant. Avantageusement, la teneur en solvant de cette fraction de tête est supérieure ou égale à 95% poids, de préférence supérieure à 98% poids et de manière préférée supérieure ou égale à 99,5% poids.

### Etape c)

Conformément à l'invention, au moins une partie de la fraction de tête (31) issue de l'étape b) est soumise à une étape c) dans laquelle on sépare dans une colonne de distillation finale (32) ladite fraction comprenant essentiellement de l'hexène-1 et du butène-1 issue de l'étape b) en une fraction de tête comprenant principalement du butène-1 (33) et en une fraction de fond comprenant principalement de l'hexène-1 (34). De manière avantageuse selon l'invention, ladite colonne de distillation de l'étape de séparation c) est mise en oeuvre à une pression comprise entre 0,5 et 1,5 MPa, de préférence entre 0,7 et 1,2 MPa, une température de tête de colonne comprise entre 30 et 130°C, de préférence entre 50 et 90°C, et une température de fond de colonne comprise entre 100 et 300°C, de préférence entre 130 et 200°C.

### Description détaillée des figures

Pour une meilleure compréhension, plusieurs modes de réalisation du procédé de l'invention sont illustrés par les Figures 1 et 2. Ces modes de réalisation sont donnés à titre d'exemples et ne présentent aucun caractère limitatif. Ces illustrations du procédé de l'invention ne comportent pas les détails de l'ensemble des composants nécessaires à sa mise en oeuvre. Seuls les éléments nécessaires à la compréhension de l'invention y sont représentés, l'homme du métier étant en capacité de compléter ces représentations pour réaliser et mettre en oeuvre l'invention.

La Figure 1 illustre, de manière non limitative, un mode de réalisation, dans lequel au moins une partie de la fraction de fond issue de l'étape a) est envoyée dans une colonne de distillation pour en éliminer une fraction en C8+ avant d'être envoyée dans la colonne de distillation de l'étape b).

L'alimentation de la charge d'éthylène frais est réalisée par une conduite 1 vers un réacteur 12 de trimérisation de l'éthylène. Selon la figure 1, l'hydrogène 4 et/ou de l'éthylène frais 1 est(sont) mélangé(s) avec l'ethylène 7 au niveau de l'interétage 9 des compresseurs de recycle (8, 10) ou à l'aspiration 7 du premier étage du compresseur 8. Le catalyseur et l'activateur préparés et stockés dans le ballon 15 sont injectés dans le réacteur 12 par la conduite 16, plus précisément au niveau de la boucle de recyclage du réacteur (18b, 18).

Le produit de la réaction de trimérisation de l'éthylène est évacué par le fond du réacteur 12 par une conduite 21, pour être envoyé dans une unité de séparation 23. Le fond du réacteur 12, notamment le catalyseur activé, est neutralisé par l'injection d'un inhibiteur 22 dans la conduite 21. Le mélange est généralement réalisé en utilisant un mélangeur dynamique en ligne. Dans l'unité de séparation 23, on évacue dans la fraction de fond 38 le catalyseur neutralisé ainsi que les sous-produits lourds (C12+). La fraction comportant l'éthylène non converti, les autres produits formés et le solvant, est envoyée sous forme vapeur par la conduite 24 vers la première colonne de distillation 25 de l'étape a). Cette étape de séparation référencée 23 est généralement constituée par un ou plusieurs flashs (ballons-séparateurs avec équilibre liquide-vapeur) successifs avec des étapes de chauffage intermédiaires au moyen d'échangeurs de chaleur.

D'une manière générale et indépendamment de la figure 1, la fraction vapeur issue de l'unité de séparation 23 (conduite 24) est séparée dans la première colonne de distillation 25 en une fraction de tête 7 comprenant essentiellement l'éthylène non converti et une fraction de fond comprenant les oléfines en C4+. D'une manière générale et indépendamment de la figure 1, la fraction de fond issu de la colonne de distillation 25 est ensuite envoyée par le conduit 26 directement vers une colonne de distillation 30 dans laquelle on sépare d'une part une fraction de tête 31 comprenant essentiellement de l'hexène-1 et du butène et d'autre part une fraction de fond 35 comprenant le reste des produits et le solvant.

Dans la variante selon l'invention représentée sur la figure 1, l'effluent provenant de la fraction de fond issue de la colonne de distillation 25 est d'abord envoyée dans une autre colonne de distillation 27 intermédiaire dans laquelle on sépare une fraction de tête 28 (qui est envoyée vers la colonne de distillation 30) et une fraction de fond comprenant les produits en C8+ que l'on évacue par une conduite 29. Cette colonne intermédiaire n'est pas indispensable à l'invention, mais est une variante de réalisation du procédé de l'invention avantageuse dans certains cas d'application.

Au moins une partie de l'effluent provenant de la fraction de fond issue de la colonne 30 est renvoyée par une conduite 36 en tête de la première colonne de distillation 25. Au moins une partie de l'effluent provenant de la fraction de fond issue de la colonne 30 est renvoyée par la conduite 37 dans le réacteur, plus précisément dans la boucle de recyclage du réacteur (18b, 18). Au moins une autre partie de l'effluent provenant de la fraction de fond issue de la colonne 30 est utilisée dans une boucle de recirculation (conduites 19, 20) pour refroidir la section réactionnelle par l'intermédiaire de l'échangeur 17. La partie vaporisée de la fraction de recirculation (sortant de l'échangeur 17) est renvoyée dans la colonne 30 pour la rebouillir. La fraction de tête 31 issue de la colonne de distillation 30 est envoyée dans une colonne de distillation finale 32 dans laquelle on sépare une fraction de tête 33 comprenant essentiellement du butène-1 et une fraction de fond comprenant essentiellement le produit final recherché, à savoir l'hexène-1, évacué par la conduite 34.

La figure 2 représente une autre variante de réalisation du procédé de l'invention dans laquelle il n'y a pas de colonne de distillation intermédiaire (référencé 27 sur la figure 1). Dans la colonne 30, on sépare d'une part une fraction de tête 31 comprenant essentiellement de l'hexène-1 et du butène et d'autre part une fraction de fond 35 comprenant le reste des produits et le solvant. Au moins une partie de l'effluent provenant de la fraction de fond issue de la colonne 30 est utilisée dans une boucle de recirculation (conduites 19, 20) pour refroidir la section réactionnelle 12 par l'intermédiaire de l'échangeur 17. La partie vaporisée de la fraction de recirculation (sortant de l'échangeur 17) est renvoyée dans la colonne 30 pour la rebouillir.

Au moins une autre partie de l'effluent provenant de la fraction de fond issue de la colonne de distillation 30 est renvoyée par la conduite 36 dans la première colonne de distillation 25. La fraction de tête 31 issue de la colonne de distillation 30 est envoyée dans une colonne de distillation finale 32 dans laquelle on sépare une fraction de tête 33 comprenant essentiellement du butène-1 et une fraction de fond comprenant essentiellement le produit final recherché, à savoir l'hexène-1, évacué par la conduite 34.

Une troisième colonne de distillation 40 reçoit par la conduite 39 une autre partie de l'effluent provenant de la fraction de fond issue de la colonne de distillation 30 (35). La fraction de tête issue de la colonne 40 (essentiellement composée du solvant) est renvoyée par une conduite 41 dans le réacteur 12, plus précisément dans la boucle de recyclage du réacteur (18b, 18). La fraction de fond issue de la colonne 40 comprenant les oléfines en C8+ est évacuée par la conduite 42.

Les exemples suivants illustrent la présente invention sans toutefois en limiter la portée.

### Exemples

### Exemple 1 (selon l'invention) :

Le schéma de procédé mis en oeuvre est celui de la figure 1.
La charge 1 utilisée est composée d'éthylène de qualité polymère (polymer grade selon la terminologie anglo-saxonne, soit environ 99,9% d'éthylène). Le système catalytique utilisé dans le réacteur d'oligomérisation 12 comprend les éléments suivants : tris (éthyl-2-hexanoate) de chrome, bis (2,6-diphényl-phénoxy) magnésium, et triéthyl aluminium dans les proportions molaires suivantes : 1/1/3. L'inhibiteur utilisé est de l'éthyl-2 hexanol. Ces éléments constitutifs du catalyseur sont mis en contact dans du cyclohexane séché et désaéré (solvant). Le réacteur est opéré à 135°C et au point de bulle. La température du réacteur est maintenue constante. La chaleur de la réaction est éliminée grâce à la boucle de recyclage (18,18b) et à l'échangeur 17 qui permet d'échanger de la chaleur avec la boucle de recirculation (19, 20).

La colonne 25 est opérée, selon les règles connues de l'homme de l'art, de manière à séparer l'éthylène résiduel du solvant et des produits de la réaction.

La colonne 27 permet de séparer en fond de colonne une coupe contenant les oléfines en C8+ et en tête de colonne le solvant, l'hexène-1 et le butène-1. Elle est opérée à une pression de 0,23 MPa en tête de colonne, une température de tête de colonne de 108°C et une température de fond de colonne de 190 °C.

La colonne 30 permet de séparer le butène-1 et l'hexène-1 du solvant. Elle est opérée avec une température de tête de colonne de 75°C et une température de fond de colonne de 110°C. La pression en tête de colonne est 0,15 MPa.

La colonne 32 permet de séparer en fond de colonne l'hexène-1 produit et en tête de colonne une coupe riche en butène-1. Elle est opérée à une pression de 1,06 MPa en tête de colonne, une température de tête de colonne de 73 °C et une température de fond de colonne de 165 °C.

Après séparation dans les colonnes 25, 27, 30, 32, on produit 0,86 tonnes d'hexène-1 (34), 0,015 tonnes d'une coupe riche en butène-1 (33) et 0,066 tonnes d'une coupe en C8+ (29) par tonne d'éthylène introduit par le conduit 1.

1,12 tonnes de vapeur basse pression (0,4 MPa) par tonne de charge sont nécessaires pour opérer le rebouillage de la colonne 30.

### Exemple 2 (comparatif) :

Comme dans l'exemple 1, le solvant utilisé est le cyclohexane.
Le procédé mis en oeuvre se distingue de celui mis en oeuvre à l'exemple 1 par le fait que l'échangeur 17 n'est pas alimenté par le solvant séparé à la colonne de distillation 30, mais par de l'eau. Il n'y a donc pas de retour du solvant après échange vers la colonne 30.

L'échangeur 17 est alimenté par de l'eau qui est vaporisée. De la vapeur très basse pression (0,17 MPa) est ainsi produite qui ne peut pas être valorisée dans une autre partie du procédé. Cette vapeur est donc condensée, pompée et recyclée vers l'échangeur 17.

Les colonnes 25, 27, 30 et 32 sont opérées dans les mêmes conditions que dans l'exemple 1.

Après séparation dans les colonnes 25, 27, 30, 32, on produit 0, 86 tonnes d'hexène-1 (34), 0,015 tonnes d'une coupe riche en butène-1 (33) et 0,066 tonnes d'une coupe en C8+ (29) par tonne de charge (éthylène).

1,75 tonnes de vapeur basse pression (0,4 MPa) par tonne de charge sont nécessaires pour opérer le rebouillage de la colonne 30. Par rapport au procédé selon l'invention de l'exemple 1, il apparait que 0,63 tonnes supplémentaires de vapeur basse pression (0,4 MPa) par tonne de charge sont nécessaires.

### Exemple 3 (selon l'invention) :

Le procédé utilisé diffère de celui de l'exemple 1 en ce que le solvant utilisé est le 2,2 diméthyl-pentane.

La colonne 25 est opérée, selon les règles connues de l'homme de l'art, de manière à séparer l'éthylène résiduel du solvant et des produits de la réaction.

La colonne 27 est opérée à une pression de 0,23 MPa en tête de colonne, une température de tête de colonne de 107 °C et une température de fond de colonne de 191 °C.

La colonne 30 est opérée avec une température de tête de colonne de 76 °C et une température de fond de colonne de 108 °C. La pression en tête de colonne est 0,15 MPa.

La colonne 32 est opérée à une pression de 1,06 MPa en tête de colonne, une température de tête de colonne de 73 °C et une température de fond de colonne de 165. °C.

Après séparation dans les colonnes 25, 27, 30, 32, on produit 0,86 tonnes d'hexène-1 (34), 0,015 tonnes d'une coupe riche en butène-1 (33) et 0,066 tonnes d'une coupe en C8+ (29) par tonne d'éthylène introduit par le conduit.

1,04 tonnes de vapeur basse pression (0,4 MPa) par tonne de charge sont nécessaires pour opérer le rebouillage de la colonne 30.

### Exemple 4 (comparatif)

Comme dans l'exemple 3, le solvant utilisé est le 2,2-diméthyl-pentane
Le procédé mis en oeuvre se distingue de celui mis en oeuvre à l'exemple 3 par le fait que l'échangeur 17 n'est pas alimenté par le solvant séparé à la colonne de distillation 30, mais par de l'eau. Il n'y a donc pas de retour du solvant après échange vers la colonne 30.

L'échangeur 17 est alimenté par de l'eau qui est vaporisée. De la vapeur très basse pression (0,17 MPa) est ainsi produite qui ne peut pas être valorisée dans une autre partie du procédé. Cette vapeur est donc condensée, pompée et recyclée vers l'échangeur 17.

Les colonnes 25, 27, 30 et 32 sont opérées dans les mêmes conditions que dans l'exemple 3.

Après séparation dans les colonnes 25, 27, 30, 32, on produit 0,86 tonnes d'hexène-1 (34), 0,015 tonnes d'une coupe riche en butène-1 (33) et 0,066 tonnes d'une coupe en C8+ (29) par tonne de charge (éthylène).

1,67 tonnes de vapeur basse pression (0,4 MPa) par tonne de charge sont nécessaires pour opérer le rebouillage de la colonne 30. Par rapport au procédé selon l'invention de l'exemple 3, il apparait que 0,63 tonnes supplémentaires de vapeur basse pression (0,4 MPa) par tonne de charge sont nécessaires.

### Exemple 5 (selon l'invention) :

Le schéma de procédé mis en oeuvre est celui de la figure 1.
La charge 1 utilisée est composée d'éthylène de qualité polymère (polymer grade selon la terminologie anglo-saxonne, soit environ 99,9% d'éthylène). Le système catalytique utilisé dans le réacteur d'oligomérisation 12 comprend les éléments suivants : tris(éthyl-2-hexanoate) de chrome, bis(tertiobutyl-2-phényl-6-phénoxy)magnésium, et triéthyl aluminium dans les proportions molaires suivantes : 1/1/3. L'inhibiteur utilisé est de l'éthyl-2 hexanol. Ces éléments constitutifs du catalyseur sont introduits dans du cyclohexane séché et désaéré (solvant). Le réacteur est opéré à 135 °C et au point de bulle. La température du réacteur est maintenue constante. La chaleur de la réaction est éliminée grâce à la boucle de recyclage (18,18b) et à l'échangeur 17 qui permet d'échanger de la chaleur avec la boucle de recirculation (19, 20).

La colonne 25 est opérée, selon les règles connues de l'homme de l'art, de manière à séparer l'éthylène résiduel du solvant et des produits de la réaction.

La colonne 27 permet de séparer en fond de colonne une coupe contenant les oléfines en C8+ et en tête de colonne le solvant, l'hexène-1 et le butène-1. Elle est opérée à une pression de 0,23 MPa en tête de colonne, une température de tête de colonne de 108 °C et une température de fond de colonne de 190 °C.

La colonne 30 permet de séparer le butène-1 et l'hexène-1 du solvant. Elle est opérée avec une température de tête de colonne de 75 °C et une température de fond de colonne de 110 °C. La pression en tête de colonne est 0,15 MPa.

La colonne 32 permet de séparer en fond de colonne l'hexène-1 produit et en tête de colonne une coupe riche en butène-1. Elle est opérée à une pression de 1,06 MPa en tête de colonne, une température de tête de colonne de 73 °C et une température de fond de colonne de 165 °C.

Après séparation dans les colonnes 25, 27, 30, 32, on produit 0,87 tonnes d'hexène-1 (34), 0,007 tonnes d'une coupe riche en butène-1 (33) et 0,064 tonnes d'une coupe en C8+ (29) par tonne de charge (éthylène).

1,13 tonnes de vapeur basse pression (0,4 MPa) par tonne de charge sont nécessaires pour opérer le rebouillage de la colonne 30.

### Exemple 6 (comparatif) :

Comme dans l'exemple 5, le solvant utilisé est le cyclohexane.
Le procédé mis en oeuvre se distingue de celui mis en oeuvre à l'exemple 5 par le fait que l'échangeur 17 n'est pas alimenté par le solvant séparé à la colonne de distillation 30, mais par de l'eau. Il n'y a donc pas de retour du solvant après échange vers la colonne 30.

L'échangeur 17 est alimenté par de l'eau qui est vaporisée. De la vapeur très basse pression (0,17 MPa) est ainsi produite qui ne peut pas être valorisée dans une autre partie du procédé. Cette vapeur est condensée, pompée et recyclée vers l'échangeur 17.

Les colonnes 25, 27, 30 et 32 sont opérées dans les mêmes conditions que dans l'exemple 5.

Après séparation dans les colonnes 25, 27, 30, 32, on produit 0,87 tonnes d'hexène-1 (34), 0,007 tonnes d'une coupe riche en butène-1 (33) et 0,064 tonnes d'une coupe en C8+ (29) par tonne de charge (éthylène).

1,76 tonnes de vapeur basse pression (0,4 MPa) par tonne de charge sont nécessaires pour opérer le rebouillage de la colonne 30. Par rapport au procédé selon l'invention de l'exemple 5, il apparait que 0,63 tonnes supplémentaires de vapeur basse pression (0,4 MPa) par tonne de charge sont nécessaires.

### Exemple 7 (selon l'invention) :

Le procédé utilisé diffère de celui de l'exemple 5 en ce que le solvant utilisé est le toluène.

Le réacteur est opéré à 165 °C et au point de bulle. La température du réacteur est maintenue constante.

La colonne 25 est opérée, selon les règles connues de l'homme de l'art, de manière à séparer l'éthylène résiduel du solvant et des produits de la réaction

La colonne 27 est opérée à une pression de 0,23 MPa en tête de colonne, une température de tête de colonne de 136 °C et une température de fond de colonne de 205 °C.

La colonne 30 est opérée avec une température de tête de colonne de 76°C et une température de fond de colonne de 140 °C. La pression en tête de colonne est 0,15 MPa.

La colonne 32 est opérée à une pression de 1,06 MPa en tête de colonne, une température de tête de colonne de 73 °C et une température de fond de colonne de 165 °C.

Après séparation dans les colonnes 25, 27, 30, 32, on produit 0,87 tonnes d'hexène-1 (34), 0,007 tonnes d'une coupe riche en butène-1 (33) et 0,064 tonnes d'une coupe en C8+ (29) par tonne de charge (éthylène).

Aucune addition de vapeur basse pression n'est nécessaire pour opérer le rebouillage de la colonne 30.

### Exemple 8 (comparatif)

Comme dans l'exemple 7, le solvant utilisé est le toluène
Le procédé mis en oeuvre se distingue de celui mis en oeuvre à l'exemple 7 par le fait que l'échangeur 17 n'est pas alimenté par le solvant séparé à la colonne de distillation 30, mais par de l'eau. Il n'y a donc pas de retour du solvant après échange vers la colonne 30.

L'échangeur 17 est alimenté par de l'eau qui est vaporisée. De la vapeur très basse pression (0,17 MPa) est ainsi produite qui ne peut pas être valorisée dans une autre partie du procédé. Cette vapeur est donc condensée, pompée et recyclée vers l'échangeur 17.

Le réacteur est opéré à 165 °C et au point de bulle. La température du réacteur est maintenue constante.

La colonne 25 est opérée, selon les règles connues de l'homme de l'art, de manière à séparer l'éthylène résiduel du solvant et des produits de la réaction

La colonne 27 est opérée à une pression de 0,23 MPa en tête de colonne, une température de tête de colonne de 136 °C et une température de fond de colonne de 205 °C.

La colonne 30 est opérée avec une température de tête de colonne de 76°C et une température de fond de colonne de 140 °C. La pression en tête de colonne est 0,15 MPa.

La colonne 32 est opérée à une pression de 1,06 MPa en tête de colonne, une température de tête de colonne de 73 °C et une température de fond de colonne de 165 °C.

Après séparation dans les colonnes 25, 27, 30, 32, on produit 0,87 tonnes d'hexène-1 (34), 0,007 tonnes d'une coupe riche en butène-1 (33) et 0,064 tonnes d'une coupe C8+ (29) par tonne de charge (éthylène).

0,61 tonnes de vapeur basse pression (0,4 MPa) par tonne de charge sont nécessaires pour opérer le rebouillage de la colonne 30. Par rapport au procédé selon l'invention de l'exemple 7, il apparait que 0,61 tonnes supplémentaires de vapeur basse pression (0,4 MPa) par tonne de charge sont nécessaires.

## Revendications

1. Procédé de séparation de l'hexène-1 à partir d'un mélange issu d'une section réactionnelle de trimérisation de l'éthylène, ledit mélange comprenant, l'éthylène, le solvant, le catalyseur de la trimérisation de l'éthylène et les produits formés dont l'hexène-1, le procédé comprenant au moins les étapes suivantes:
a) on sépare dans une première colonne de distillation, le mélange issu de la réaction de trimérisation de l'éthylène, en une fraction de tête comprenant l'éthylène et une fraction de fond,
b) on sépare dans au moins une autre colonne de distillation au moins une partie d'un effluent provenant de la fraction de fond issue de l'étape a) en une fraction de tête comprenant de l'hexène-1 et du butène-1 et une fraction de fond,
c) on sépare dans une colonne de distillation finale au moins une partie de la fraction comprenant de l'hexène-1 et du butène-1 issue de l'étape b) en une fraction de tête comprenant principalement du butène-1 et en une fraction de fond comprenant principalement de l'hexène-1 et dans ledit procédé :
- au moins une partie d'un effluent provenant de la fraction de fond issue de l'étape b) est renvoyée dans la section réactionnelle et au moins une autre partie de ladite fraction de fond issue de l'étape b) est utilisée dans au moins une boucle de recirculation reliant la section réactionnelle et la colonne de ladite étape b), ladite boucle de recirculation permettant de refroidir la section réactionnelle et de rebouillir ladite colonne de l'étape b).

2. Procédé selon la revendication 1 dans lequel au moins une partie d'un effluent provenant de la fraction de fond issue de l'étape b) est envoyée dans au moins une colonne de distillation pour en éliminer une fraction de fond comprenant essentiellement des oléfines en C8+ avant de renvoyer au moins une partie de la fraction de tête issue de ladite colonne de distillation dans la section réactionnelle.

3. Procédé selon la revendication 1 dans lequel au moins une partie d'un effluent provenant de la fraction de fond issue de l'étape a) est envoyée dans au moins une colonne de distillation pour en éliminer une fraction de fond comprenant essentiellement des oléfines en C8+ avant d'envoyer au moins une partie de la fraction de tête issue de ladite colonne de distillation dans la colonne de distillation de l'étape b).

4. Procédé selon l'une des revendications précédentes dans lequel on renvoie au moins une partie d'un effluent provenant de la fraction de fond issue de l'étape b) en tête de la première colonne de l'étape a) pour laver l'éthylène sortant en tête de ladite première colonne et entrainer la fraction d'oléfines en C4⁺ vers le fond de ladite première colonne.

5. Procédé selon l'une des revendications précédentes dans lequel l'éthylène non réagi séparé de la première colonne de distillation de l'étape a) est renvoyé dans la section réactionnelle.

6. Procédé selon la revendication 5 dans lequel de l'éthylène frais est mélangé avec l'éthylène renvoyé dans la section réactionnelle.

7. Procédé selon les revendications 5 ou 6 dans lequel de l'hydrogène est mélangé avec l'éthylène non réagi renvoyé dans la section réactionnelle.

8. Procédé selon l'une des revendications précédentes dans lequel la section réactionnelle comprend au moins un réacteur de trimérisation.

9. Procédé selon la revendication 8 dans lequel les conditions opératoires du réacteur de trimérisation sont telles que le milieu réactionnel est au point de bulle.

10. Procédé selon la revendication 9 tel qu'on soutire de manière permanente de la vapeur (13) en tête de réacteur de trimérisation.

11. Procédé selon la revendication 10 tel qu' au moins une partie de la vapeur soutirée en tête de réacteur (13) est envoyée (14) dans la colonne de distillation de l'étape a).

12. Procédé selon l'une des revendications précédentes dans lequel la réaction de trimérisation de l'éthylène est opérée à une température comprise entre 0 et 300 °C et une pression comprise entre 0,1 et 35 MPa.

13. Procédé selon l'une des revendications précédentes comprenant une étape dans laquelle on neutralise le catalyseur contenu dans le mélange issu de la section réactionnelle de trimérisation de l'éthylène par l'injection d'un inhibiteur.

14. Procédé selon la revendication 13 dans lequel au moins une partie de la fraction de fond (21) neutralisée est envoyée dans une unité de séparation (23) dans laquelle on sépare d'une part le catalyseur neutralisé ainsi que les sous-produits lourds (C12+) de la réaction et d'autre part l'éthylène non converti, les autres produits formés et le solvant.

15. Procédé selon la revendication 14 tel que la séparation est réalisée par une série de flashs étagés.

## Patentansprüche

1. Verfahren zur Trennung des Hexen-1 aus einer Mischung, die aus einem Trimerisierungsabschnitt des Ethylens stammt, wobei die Mischung das Ethylen, das Lösungsmittel, den Katalysator der Trimerisierung des Ethylens und die gebildeten Produkte, unter anderem das Hexen-1, umfasst, wobei das Verfahren mindestens die folgenden Schritte umfasst:
a) man trennt in einer ersten Destillationskolonne die aus der Trimerisierungsreaktion des Ethylens stammenden Mischung in eine Kopffraktion, umfassend das Ethylen, und eine Bodenfraktion,
b) man trennt in mindestens einer weiteren Destillationskolonne mindestens einen Teil eines aus der Bodenfraktion aus Schritt a) stammenden Abstrom in eine Kopffraktion, umfassend Hexen-1 und Buten-1, und eine Bodenfraktion,
c) man trennt in einer End-Destillationskolonne mindestens einen Teil der Hexen-1 und Buten-1 umfassenden Fraktion aus Schritt b) in eine Kopffraktion, umfassend hauptsächlich Buten-1 und eine Bodenfraktion, umfassend hauptsächlich Hexen-1,
und wobei bei dem Verfahren:
- mindestens ein Teil eines von der Bodenfraktion aus Schritt b) stammenden Abstrom wieder in den Reaktionsabschnitt zurückgeschickt wird, und mindestens ein weiterer Teil der aus Schritt b) stammenden Bodenfraktion in mindestens einer Rezirkulationsschleife verwendet wird, die den Reaktionsabschnitt und die Kolonne des Schritts b) verbindet, wobei es die Rezirkulationsschleife ermöglicht, den Reaktionsabschnitt zu kühlen und die Kolonne aus Schritt b) erneut zum Sieden zu bringen.

2. Verfahren nach Anspruch 1, bei dem mindestens ein Teil eines von der Bodenfraktion aus Schritt b) stammenden Abstroms in mindestens eine Destillationskolonne geschickt wird, um daraus eine Bodenfraktion, umfassend im Wesentlichen Olefine C8+, zu beseitigen, bevor mindestens ein Teil der aus der Destillationskolonne stammenden Kopffraktion in den Reaktionsabschnitt zurückgeschickt wird.

3. Verfahren nach Anspruch 1, bei dem mindestens ein Teil eines von der Bodenfraktion aus Schritt a) stammenden Abstroms in mindestens eine Destillationskolonne geschickt wird, um daraus eine Bodenfraktion, umfassend im Wesentlichen Olefine C8+, zu beseitigen, bevor mindestens ein Teil der aus der Destillationskolonne stammenden Kopffraktion in die Destillationskolonne aus Schritt b) zurückgeschickt wird.

4. Verfahren nach einem der vorhergehenden Ansprüche, bei dem mindestens ein Teil eines von der Bodenfraktion aus Schritt b) stammenden Abstroms am Kopf der ersten Kolonne des Schrittes a) zurückgeschickt wird, um das am Kopf der ersten Kolonne austretende Ethylen zu waschen und die Fraktion von Olefinen C4⁺ zum Boden der ersten Kolonne mitzunehmen.

5. Verfahren nach einem der vorhergehenden Ansprüche, bei dem das nicht reagierte, von der ersten Destillationskolonne aus Schritt a) getrennte Ethylen in den Reaktionsabschnitt zurückgeschickt wird.

6. Verfahren nach Anspruch 5, bei dem das frische Ethylen mit dem in den Reaktionsabschnitt zurückgeschickten Ethylen gemischt wird.

7. Verfahren nach den Ansprüchen 5 oder 6, bei dem Wasserstoff mit dem nicht reagierten, in den Reaktionsabschnitt zurückgeschickten Ethylen gemischt wird.

8. Verfahren nach einem der vorhergehenden Ansprüche, bei dem der Reaktionsabschnitt mindestens einen Trimerisierungsreaktor umfasst.

9. Verfahren nach Anspruch 8, bei dem die Betriebsbedingungen des Trimerisierungsreaktors derart sind, dass das Reaktionsmedium am Blasenpunkt ist.

10. Verfahren nach Anspruch 9, bei dem permanent Dampf (13) am Kopf des Trimerisierungsreaktors entnommen wird.

11. Verfahren nach Anspruch 10, bei dem mindestens ein Teil des am Reaktorkopfes (13) entnommenen Dampfes in die Destillationskolonne aus Schritt a) zurückgeschickt wird (14).

12. Verfahren nach einem der vorhergehenden Ansprüche, bei dem die Trimerisierungsreaktion des Ethylens bei einer Temperatur zwischen 0 und 300°C und einem Druck zwischen 0,1 und 35 MPa durchgeführt wird.

13. Verfahren nach einem der vorhergehenden Ansprüche, umfassend einen Schritt, bei dem der Katalysator, der in der Mischung enthalten ist, die aus dem Reaktionsabschnitt einer Trimerisierung des Ethylens stammt, durch das Einspritzen eines Inhibitors neutralisiert wird.

14. Verfahren nach Anspruch 13, bei dem mindestens ein Teil der neutralisierten Bodenfraktion (21) in eine Trennungseinheit (23) geschickt wird, in der einerseits der neutralisierte Katalysator sowie die schweren Unterprodukte (C12+) der Reaktion und andererseits das nicht konvertierte Ethylen, die andere geformten Produkte und das Lösungsmittel getrennt werden.

15. Verfahren nach Anspruch 14, bei dem die Trennung durch eine Reihe von gestuften Flashs erfolgt.

## Claims

1. A process for separating 1-hexene from a mixture obtained from an ethylene trimerization reaction section, said mixture comprising ethylene, solvent, ethylene trimerization catalyst and products formed, including 1-hexene, the process comprising at least the following steps:
a) separating the mixture obtained from the ethylene trimerization reaction in a first distillation column into a top fraction comprising ethylene and a bottom fraction;
b) separating, in at least one other distillation column, at least a portion of an effluent deriving from the bottom fraction obtained from step a) into a top fraction comprising 1-hexene and 1-butene and a bottom fraction;
c) separating, in a final distillation column, at least a portion of the fraction comprising 1-hexene and 1-butene obtained from step b) into a top fraction principally comprising 1-butene and into a bottom fraction principally comprising 1-hexene, and in said process:
- at least a portion of an effluent deriving from the bottom fraction obtained from step b) is returned to the reaction section and at least one other portion of said bottom fraction obtained from step b) is used in at least one recirculation loop connecting the reaction section and the column of step b), said recirculation loop permit to obtain cooling of the reaction section and the reboiling of said column.

2. A process according to claim 1, in which at least a portion of an effluent deriving from the bottom fraction obtained from step b) is sent to at least one distillation column in order to eliminate a bottom fraction essentially comprising C8+ olefins therefrom before returning at least a portion of the top fraction obtained from said distillation column to the reaction section.

3. A process according to claim 1, in which at least a portion of an effluent deriving from the bottom fraction obtained from step a) is sent to at least one distillation column in order to eliminate a bottom fraction essentially comprising C8+ olefins therefrom before sending at least a portion of the top fraction obtained from said distillation column to the distillation column of step b).

4. A process according to one of the preceding claims, in which at least a portion of an effluent deriving from the bottom fraction obtained from step b) is returned to the top of the first column of step a) in order to wash the ethylene leaving from the top of said first column and to entrain the C4+ olefins fraction towards the bottom of said first column.

5. A process according to one of the preceding claims, in which the unreacted ethylene separated from the first distillation column of step a) is returned to the reaction section.

6. A process according to claim 5, in which fresh ethylene is mixed with the ethylene returned to the reaction section.

7. A process according to claim 5 or claim 6, in which hydrogen is mixed with the unreacted ethylene returned to the reaction section.

8. A process according to one of the preceding claims, in which the reaction section comprises at least one trimerization reactor.

9. A process according to claim 8, in which the operating conditions for the trimerization reactor are such that the reaction medium is at the bubble point.

10. A process according to claim 9, in which vapour (13) is continuously withdrawn from the top of the trimerization reactor.

11. A process according to claim 10, in which at least a portion of the vapour withdrawn from the top of the reactor (13) is sent (14) to the distillation column of step a).

12. A process according to one of the preceding claims, in which the ethylene trimerization reaction is operated at a temperature in the range 0°C to 300°C and a pressure in the range 0.1 to 35 MPa.

13. A process according to one of the preceding claims, comprising a step in which the catalyst contained in the mixture obtained from the ethylene trimerization reaction section is neutralized by injecting an inhibitor.

14. A process according to claim 13, in which at least a portion of the neutralized bottom fraction (21) is sent to a separation unit (23) in which on the one hand the neutralized catalyst as well as the heavy by-products (C12+) of the reaction are separated from unconverted ethylene, the other products formed and the solvent on the other hand.

15. A process according to claim 14, in which the separation is carried out in a series of flash stages.
